# EUROPEAN PATENT SPECIFICATION

(11) **EP 4 275 724 B1**
(45) Date of publication and mention of the grant of the patent: **22.07.2026**
(21) Application number: 22752765.2
(22) Date of filing: 09.02.2022
(51) Int. Cl.: A61M 11/00

(54) **DISCHARGE DEVICE**
AUSTRAGVORRICHTUNG
DISPOSITIF DE DÉCHARGE

(30) Priority: 12.02.2021 JP 2021020489
(43) Date of publication of application: 15.11.2023
(73) Proprietor: Daikin Industries, Ltd., Osaka-shi, Osaka 530-0001 (JP)
(72) Inventor: ARAI, Junichiro, Osaka-shi, Osaka 530-8323 (JP); TAKEDA, Nobuaki, Osaka-shi, Osaka 530-8323 (JP); OZAWA, Satoshi, Osaka-shi, Osaka 530-8323 (JP)
(74) Representative: Hoffmann Eitle
(86) International application number: PCT/JP2022/005044
(87) International publication number: WO 2022/172942

(56) References cited:
- WO-A1-2017/149684
- WO-A1-2017/149684
- JP-A- 2004 053 102
- JP-A- 2018 514 436
- US-A- 4 710 887
- US-A- 4 710 887
- US-A1- 2005 205 090

## Description

### TECHNICAL FIELD

The present disclosure relates to an emission device.

### BACKGROUND ART

JP 2020-14535 A discloses an inhaler for administering medications to a respiratory system. The inhaler of JP 2020-14535 A includes a nozzle plate that is vibrated by a piezoelectric element to spray an inhalant. The sprayed inhalant is delivered to the user's respiratory organ via a mask-type inhaler portion that covers the user's nose and mouth.

US 4,710,887 A1 discloses an aerosol generating system for maintaining a constant concentration of aerosol in a chamber. The system includes an aerosol generator in a closed chamber. A small fan in the chamber distributes the aerosol within the chamber. A particle analyzer connected to the chamber draws aerosol samples and analyses samples to monitor at least one characteristic property of the aerosol. Typically, these are particulate concentration and particulate size. The system includes a control responsive to signals from the analyzer representing changes in the monitored characteristic property to control operation of the aerosol generator. The control is such as to maintain the characteristic property substantially constant. The system may also be equipped with humidity and temperature control elements for maintaining these factors constant within the chamber.

US 2005/0205090 A1 discloses a system comprising means for automatically controlling an environment of an inhalant chamber and means for automatically controlling a concentration of an inhalant in the inhalant chamber. Wherein the means for automatically controlling an environment of an inhalant chamber includes means for maintaining an environmental factor via feedback control, wherein the environmental factor includes a pressure of the inhalant chamber.

### SUMMARY OF THE INVENTION

### TECHNICAL PROBLEM

When a person inhales a useful substance, the ease of inhalation of the useful substance into the body varies depending on environmental conditions where the person inhales the substance. The inhaler of JP 2020-14535 A does not consider the environmental conditions where the user undergoes inhalation, and thus the useful substance is not always administered efficiently to the user.

It is an object of the present disclosure to efficiently administer a useful substance to a target person.

### SOLUTION TO THE PROBLEM

The present invention is defined by independent claim 1. The dependent claims describe optional features and particular embodiments.

A first aspect of the present disclosure is directed to an emission device including: an emission unit (30) configured to emit a useful substance (U) to a space (S) where a target person (T) is present; an environment adjustment unit (90) configured to adjust an environmental condition of the space (S); a storage (82) configured to store data on the useful substance (U) and an environmental condition associated with the useful substance (U); and a controller (80) configured to acquire the environmental condition associated with the useful substance (U) emitted from the emission unit (30), based on the data stored in the storage (82), and control the environment adjustment unit (90) to meet the environmental condition.

**In** the first aspect, the controller (80) controls the environment adjustment unit (90) to meet the environmental condition associated with the useful substance (U), based on the data stored in the storage (82). Accordingly, the information on the environmental condition in which the useful substance (U) efficiently acts on the target person (T) is stored in the storage (82), whereby the useful substance (U) can be efficiently administered to the target person (T).

A second aspect of the present disclosure is directed to the emission device of the first aspect, further including a concentration sensor (53) configured to measure a concentration of the useful substance (U) supplied to the target person (T), wherein the controller (80) adjusts an emission amount of the useful substance (U) based on an output from the concentration sensor (53).

**In** the second aspect, the concentration of the useful substance (U) supplied to the target person (T) can be maintained at an appropriate concentration by the concentration sensor (53).

A third aspect of the present disclosure is directed to the emission device of the first or second aspect, wherein the environment adjustment unit (90) adjusts a temperature of air in the space (S), and the controller (80) acquires a temperature of the space (S) associated with the useful substance (U), based on the data stored in the storage (82), and controls the environment adjustment unit (90) to meet the temperature.

**In** the third aspect, the temperature of the space (S) can be adjusted to a temperature at which the useful substance (U) easily acts on the target person (T).

A fourth aspect of the present disclosure is directed to the emission device of any one of the first to third aspect, wherein the environment adjustment unit (90) adjusts a humidity of air in the space (S), and the controller (80) acquires a humidity of the space (S) associated with the useful substance (U), based on the data stored in the storage (82), and controls the environment adjustment unit (90) to meet the humidity.

In the fourth aspect, the humidity of the space (S) can be adjusted to humidity at which the useful substance (U) easily acts on the target person (T).

A fifth aspect of the present disclosure is directed to the emission device of any one of the first to fourth aspect, wherein the environment adjustment unit (90) adjusts an airflow in the space (S), and the controller (80) acquires an airflow of the space (S) associated with the useful substance (U), based on the data stored in the storage (82), and controls the environment adjustment unit (90) to meet the airflow.

In the fifth aspect, the airflow of the space (S) can be adjusted to an airflow in which the useful substance (U) easily acts on the target person (T).

A sixth aspect of the present disclosure is directed to the emission device of any one of the first to fifth aspect, wherein the environment adjustment unit (90) emits an airflow in a vortex ring shape.

In the sixth aspect, the environment adjustment unit (90) emits an airflow in a vortex ring shape with high directivity. Thus, the useful substance (U) applied to the vortex ring (R) can be efficiently administered to the target person (T).

### BRIEF DESCRIPTION OF THE DRAWINGS

FIG. 1 is a diagram illustrating a schematic overall configuration of an emission device of a first embodiment.
FIG. 2 is a diagram illustrating a vertical cross-sectional view of the emission device.
FIG. 3 is a schematic block diagram of the emission device.
FIG. 4 is a flowchart of control of the emission device.
FIG. 5 is a flowchart of temperature control.
FIG. 6 is a flowchart of humidity control.
FIG. 7 is a flowchart of airflow control.
FIG. 8 is a flowchart of concentration control.
FIG. 9 is a flowchart corresponding to FIG. 4 of a first variation of the first embodiment.
FIG. 10 is a block diagram corresponding to FIG. 3 of a fourth variation of the first embodiment.
FIG. 11 is a diagram illustrating a schematic overall configuration of an emission device of a second embodiment.
FIG. 12 is a diagram illustrating a schematic overall configuration of an emission device of a third embodiment.

### DESCRIPTION OF EMBODIMENTS

Hereinafter, embodiments of the present disclosure will be described with reference to the drawings. The following embodiments are merely exemplary ones in nature, and are not intended to limit the scope, application, or use of the present invention.

### <<First Embodiment>>

A first embodiment will be described.

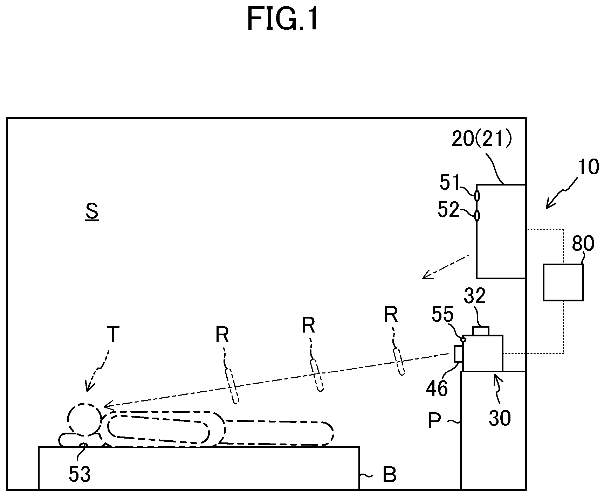

As illustrated in FIG. 1, an emission device (10) of the first embodiment is disposed in an indoor space (S) in which a target person (T) is present. The target person (T) sleeps, for example, on bedding (B) such as a bed in the indoor space (S). In this embodiment, the target person (T) has a respiratory disease of asthma and has symptoms of a cough attack.

The emission device (10) of the first embodiment is a device that emits a useful substance (U) to the target person (T). Specifically, the emission device (10) emits the useful substance (U) to a target portion of the target person (T). In this embodiment, the target portion of the target person (T) includes his/her mouth and nose.

In this embodiment, the useful substance (U) is salbutamol (short-acting β2 stimulant). The amount of components contained in the useful substance (U) is appropriately selected depending on symptoms of the target person (T), other components blended with the above components, and the like, and is not particularly limited.

### -Emission Device-

As illustrated in FIG. 1, the emission device (10) includes an air conditioning unit (20), an emission unit (30), a temperature sensor (51), a humidity sensor (52), a concentration sensor (53), a camera (55), and a controller (80).

### <Air Conditioning Unit>

The air conditioning unit (20) performs air conditioning of the indoor space (S). As illustrated in FIG. 1, the air conditioning unit (20) includes an indoor unit (21) of a wall-mounted type for example. The indoor unit (21) is coupled to an outdoor unit (not shown) via a refrigerant pipe. The air conditioning unit (20) cools or heats indoor air (room air) with a refrigerant with which a refrigeration cycle is performed. In this manner, the temperature of air in the indoor space (S) is adjusted. The air conditioning unit (20) adjusts the humidity of the indoor air (room air) in addition to the temperature thereof. The air conditioning unit (20) constitutes an environment adjustment unit (90) that adjusts the temperature and humidity which are environmental conditions of the indoor space (S).

### -Emission Unit-

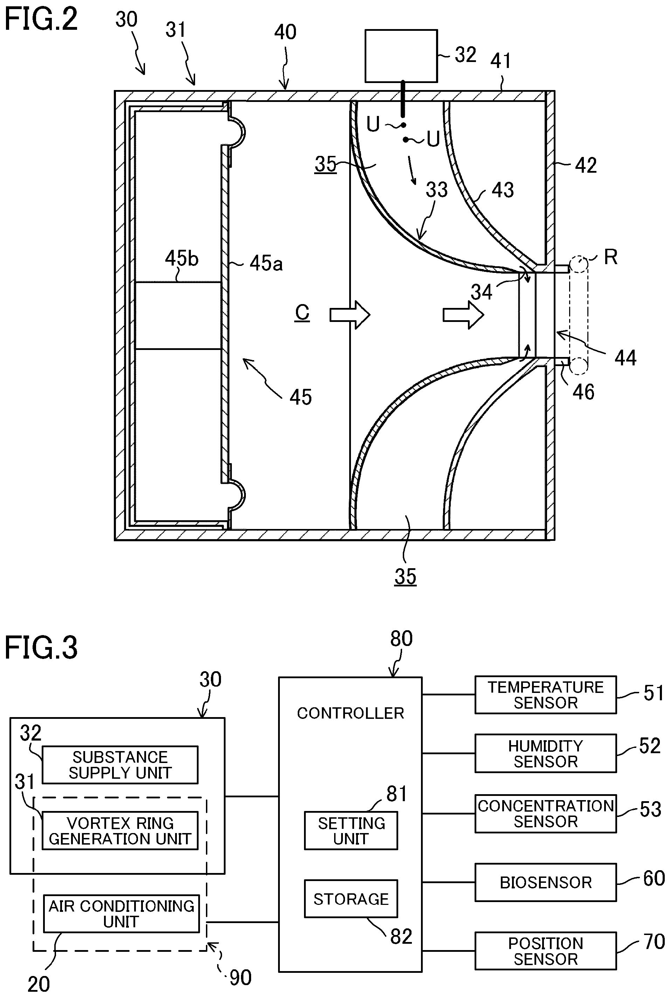

As illustrated in FIG. 1, the emission unit (30) is placed on a shelf (P) in the indoor space (S) for example. The emission unit (30) emits the useful substance (U) to the indoor space (S). As illustrated in FIG. 2, the emission unit (30) includes a vortex ring generation unit (31) and a substance supply unit (32). The vortex ring generation unit (31) emits an airflow (vortex ring (R)) in a vortex ring shape. The substance supply unit (32) supplies the useful substance (U) to the airflow (vortex ring (R)).

The emission unit (30) makes the useful substance (U) contained in the vortex ring (R) and supplies the vortex ring (R) containing the useful substance (U) toward the target portion of the target person (T). The useful substance (U) may be gas, liquid, or solid, and is preferably in particulate form if liquid or solid. The vortex ring generation unit (31) of this embodiment constitutes the environment adjustment unit (90) that adjusts the airflow which is an environmental condition of the indoor space (S).

### (Vortex Ring Generation Unit)

As illustrated in FIG. 2, the vortex ring generation unit (31) includes a casing (40), an extrusion mechanism (45), and a movable nozzle (46). The casing (40) includes a casing body (41), a front panel (42), and a peripheral wall (43). The casing body (41) has a box shape where the front side is open. The front panel (42) has a substantially plate shape to block an opening surface of the front side of the casing body (41). A center portion of the front panel (42) has an emission port (44) in a circular shape passing therethrough in a front-rear direction. The peripheral wall (43) has a substantially cylindrical shape continuous with a rear surface of the front panel. The peripheral wall (43) has a tapered shape having a diameter which is gradually decreasing frontward.

The extrusion mechanism (45) is disposed rearward in the casing (40). The extrusion mechanism (45) includes a diaphragm (45a) and a linear actuator (45b) for actuating the diaphragm (45a). The linear actuator (45b) displaces the diaphragm (45a) back and forth. A proximal end (rear end) of the linear actuator (45b) is supported by a rear wall of the casing body (41). A distal end (front end) of the linear actuator (45b) is coupled with a center of the diaphragm (45a).

In the casing (40), an air passage (C) through which air flows extends from the diaphragm (45a) to the emission port (44). In the vortex ring generation unit (31), the air in the air passage (C) extruded by the extrusion mechanism (45) is formed into the vortex ring (R) and emitted from the emission port (44).

The movable nozzle (46) is provided on a center portion of the front panel (42). A distal end of the movable nozzle (46) has a circular shape. The movable nozzle (46) is coupled with a motor (not shown) via a shaft. The motor drives and rotates the shaft to adjust the direction of the movable nozzle (46). The movable nozzle (46) is directed toward the indoor space (S). The direction of the movable nozzle (46) is adjusted to change the direction of the airflow (vortex ring (R)). This allows the vortex ring generation unit (31) to emit the vortex ring (R) toward the target portion of the target person (T).

### (Substance Supply Unit, Passage Formation Portion, and Substance Supply Port)

The substance supply unit (32) is a device for providing the airflow (vortex ring (R)) with a predetermined useful substance (U). The substance supply unit (32) includes a tank storing the useful substance (U) and a transfer unit (not shown) configured to transfer the useful substance (U) in the tank. The transfer unit is, for example, an air pump. The substance supply unit (32) may include a vaporization type substance generator that vaporizes the useful substance.

The emission unit (30) further includes a passage formation portion (33) and a substance supply port (34). The passage formation portion (33) is disposed behind the peripheral wall (43) of the casing (40). The passage formation portion (33) has a substantially cylindrical shape along an inner peripheral surface of the peripheral wall (43). The passage formation portion (33) has a tapered shape having a diameter gradually decreasing frontward.

A reservoir (35) temporarily storing the useful substance (U) is defined by an inner wall of the casing body (41), the peripheral wall (43), and the passage formation portion (33). The reservoir (35) is a substantially cylindrical space formed around the passage formation portion (33). The useful substance (U) adjusted to a predetermined concentration is supplied appropriately from the substance supply unit (32) to the reservoir (35).

The emission unit (30) includes the substance supply port (34). The substance supply port (34) is an opening for supplying the useful substance (U) to the air passage (C). The substance supply port (34) is disposed near the emission port (44). Specifically, the substance supply port (34) is provided between a downstream end of the passage formation portion (33) in the cylindrical axis direction and an inner peripheral portion of the emission port (44). Thus, the substance supply port (34) has an annular shape around a downstream end of the air passage (C). The useful substance (U) is supplied from the substance supply unit (32) to the reservoir (35) defined in the casing (40), and is supplied to the air passage (C) through the substance supply port (34).

### <Temperature Sensor and Humidity Sensor>

The temperature sensor (51) measures the temperature of the indoor space (S). The humidity sensor (52) measures the humidity of the indoor space (S). The temperature sensor (51) and the humidity sensor (52) of this embodiment are attached to the indoor unit (21) of the air conditioning unit (20). The temperature sensor (51) and the humidity sensor (52) may be attached to the emission unit (30), or may be provided in the indoor space (S) independently of the air conditioning unit (20) and the emission unit (30).

### <Concentration Sensor>

The concentration sensor (53) measures the concentration of the useful substance (U) supplied to the target person (T). The concentration sensor (53) of this embodiment is placed next to the head of the target person (T). The concentration sensor (53) of this embodiment detects the concentration of the useful substance (U) in the indoor space (S). The concentration sensor (53) may be provided on the emission unit (30) or the air conditioning unit (20), or may be adhered to the target portion of the target person (T).

### <Camera>

The camera (55) is attached to the emission unit (30) and captures an image of the indoor space (S). The camera (55) captures an image of respiration of the target person (T) to detect a respiratory cycle of the target person (T). In other words, the camera (55) serves as a biosensor (60).

In addition, the camera (55) detects the positions of the target portions (the mouth and nose) of the target person (T). In other words, the camera (55) serves as a position sensor (70) that identifies the positional information of the target portions of the target person (T).

### <Controller>

The controller (80) includes a microcomputer mounted on a control board, and a memory device (specifically, a semiconductor memory) storing software for operating the microcomputer. The controller (80) includes a storage (82) and a setting unit (81). The controller (80) may include a storage (82) separate from the control board.

The storage (82) stores data including multiple types of useful substances (U) and environmental conditions associated with each of the useful substances (U). The environmental conditions associated with the useful substance (U) mean environmental conditions in which the useful substance (U) efficiently acts on the target person (T). The environmental conditions mentioned herein include the temperature and humidity of the indoor space (S) and the target portion (the airflow direction).

The useful substance (U) stored in the storage (82) may be a single substance, or may be a mixed substance in which multiple substances are mixed. The mixed substance may be different from other mixed substances in the mixing ratio of the substances contained.

The storage (82) stores data on symptoms or diseases associated with each useful substance (U), the concentration of the useful substance (U), and emission time of the useful substance (U), as well as environmental conditions associated with the useful substance (U).

A symptom or disease selected by the target person (T) is set in the setting unit (81). When a symptom or disease is set in the setting unit (81), the controller (80) selects a predetermined useful substance (U) associated with a symptom or disease stored in the storage (82) based on the symptom or disease set in the setting unit (81).

As illustrated in FIG. 3, the controller (80) is connected to the emission unit (30), the environment adjustment unit (90), the temperature sensor (51), the humidity sensor (52), the concentration sensor (53), the biosensor (60), and the position sensor (70) in a wired or wireless manner, to be able to transmit and receive signals to and from them.

The controller (80) controls the extrusion mechanism (45) of the vortex ring generation unit (31). Thus, the vortex ring (R) containing the useful substance (U) is emitted from the vortex ring generation unit (31) of the emission unit (30). **In** addition, based on the respiratory cycle detected by the biosensor (60), the controller (80) makes the emission unit (30) emit the useful substance (U) at the time when the target person (T) inhales air.

The controller (80) acquires the environmental conditions associated with the useful substance (U) emitted from the emission unit (30), based on the data stored in the storage (82), and controls the environment adjustment unit (90) to meet the environmental conditions. Specifically, the controller (80) makes the vortex ring generation unit (31) emit the vortex ring (R) based on the data of the target portion stored in the storage (82).

At this time, the controller (80) adjusts the direction of the movable nozzle (46) of the emission unit (30) based on the data of the target portion stored in the storage (82). Thus, the vortex ring (R) is emitted toward the target portion of the target person (T). Further, based on the temperature and humidity data stored in the storage (82), the controller (80) controls the air conditioning unit (20) so that the indoor space (S) has a predetermined temperature and humidity.

Based on output from the concentration sensor (53), the controller (80) adjusts the amount of the useful substance (U) emitted from the emission unit (30). Specifically, from the concentration of the useful substance (U) in the indoor space (S) output from the concentration sensor (53), the controller (80) estimates the concentration of the useful substance (U) in the target portion based on the distance between the concentration sensor (53) and the target portion of the target person (T). The controller (80) adjusts the emission amount of the useful substance (U) so that the estimated concentration reaches a predetermined concentration stored in the storage (82). Thus, the concentration of the useful substance (U) supplied to the target person (T) can be maintained at a predetermined concentration.

The controller (80) makes the emission unit (30) emit the useful substance (U) so that the cumulative total of time during which the useful substance (U) is emitted from the emission unit (30) is one hour or more in one day. Thus, the useful substance (U) can be administered to the target person (T) over a long period of time.

The controller (80) may be provided in the emission unit (30) or may be separate from the emission unit (30). For example, the controller (80) may be provided in a control unit or a remote controller of the air conditioning unit (20). The controller (80) may be provided in a server device connected to a local network, the Internet, or the like, or various communications terminals (a portable terminal, a personal computer, or the like).

### -Operation-

The operation of the emission device (10) will be described with reference to FIGS. 1 to 8.

### (Emission Operation of Vortex Ring Generation Unit)

As illustrated in FIG. 2, when the controller (80) sends a signal to the vortex ring generation unit (31), the diaphragm (45a) of the extrusion mechanism (45) is actuated by the linear actuator (45b). When the diaphragm (45a) moves back and forth, the air in the air passage (C) is pushed out toward the emission port (44).

The substance supply port (34) is provided at an outlet end of the air passage (C). The air at the outlet end of the air passage (C) has the highest flow velocity in the air passage (C), and thus has the lowest pressure. Therefore, when the air at low pressure passes through the substance supply port (34), the useful substance (U) in the reservoir (35) is sucked into the air passage (C) due to the difference between the pressure of the air and the pressure in reservoir (35). In this way, the air containing the useful substance (U) reaches the emission port (44) immediately.

The air passing through the emission port (44) has a relatively high flow velocity, whereas the air therearound is still. For this reason, a shearing force acts on the air at the plane of discontinuity of such two types of air flows to generate a vortex flow near an outer peripheral portion of the emission port (44). The vortex flow forms a vortex ring-shaped airflow (vortex ring (R)) moving frontward from the emission port (44).

FIGS. 1 and 2 show this vortex ring (R) schematically. The vortex ring (R) containing the useful substance (U), emitted from the emission port (44), flows toward the target person (T), and hits the target portions (the mouth and nose) of the target person (T). The vortex ring (R) has properties of being less diffused and having high directivity. Thus, the vortex ring (R) containing the useful substance (U) can be reliably applied to the target portion of the target person (T).

From the emission port (44) of the emission unit (30), the vortex ring (R) is periodically or intermittently emitted depending on a vibration cycle of the diaphragm (45a). In addition, the flow velocity for the vortex ring (R) changes depending on the displacement amount of the diaphragm (45a). The controller (80) may change the emission cycle and the emission speed for the vortex ring (R) by changing the vibration cycle and the displacement amount of the diaphragm (45a).

### <Control of Emission Device>

When the target person (T) selects his/her symptom or disease, the selected symptom or disease is set in the setting unit (81) of the controller (80), and the operation of the emission device (10) is started. In this embodiment, the target person (T) selects asthma as a disease.

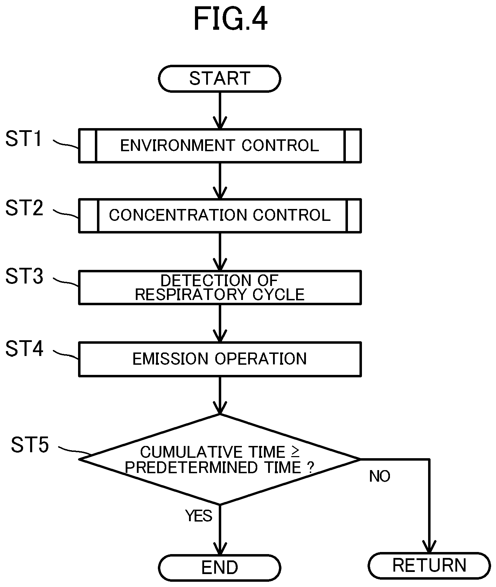

As illustrated in FIG. 4, for asthma as a disease, the controller (80) controls the environment adjustment unit (90) when the emission device (10) is in an operating state (step ST1). Specifically, the controller (80) acquires the environmental conditions associated with the predetermined useful substance (U), based on the data stored in the storage (82), and controls the environment adjustment unit (90) to meet the environmental conditions.

Thus, the information on the environmental conditions in which the predetermined useful substance (U) efficiently acts on the target person (T) is stored in the storage (82), whereby an environment in which the predetermined useful substance (U) can be efficiently administered to the target person (T) is prepared. Details of the environment adjustment unit (90) will be described later.

Next, the controller (80) adjusts the emission amount of the predetermined useful substance (U) based on an output from the concentration sensor (53) (step ST2). The concentration of the useful substance (U) in this embodiment is 0.01%. The concentration control based on the output from the concentration sensor (53) will be also described later.

Next, the controller (80) detects a respiratory cycle of the target person (T) (step ST3). Specifically, the controller (80) acquires information from the biosensor (60) in the camera (55) to detect the respiratory cycle of the target person (T).

Next, the controller (80) makes the emission unit (30) emit the predetermined useful substance (U) toward the target portion of the target person (T) according to the respiratory cycle of the target person (T) (step ST4). Specifically, the controller (80) allows the vortex ring generation unit (31) of the emission unit (30) to generate an airflow (vortex ring (R)) toward the mouth and nose of the target person (T).

In this way, the predetermined useful substance (U) is emitted to the mouth and nose of the target person (T), whereby the predetermined useful substance (U) acts on the nasal mucosa and respiratory mucosa of the target person (T) and is absorbed into the body. Then, the concentration of the useful substance (U) in the blood increases. Consequently, the symptom of cough attack of the target person (T) is suppressed. In addition, the vortex ring (R) is emitted according to the respiratory cycle, and thus the amount of the useful substance (U) used can be reduced.

Next, the controller (80) determines whether or not the cumulative total of time during which the predetermined useful material (U) is emitted from the emission unit (30) is more than or equal to a predetermined time in one day (step ST5). The predetermined time in this embodiment is one hour. Thus, the predetermined useful substance (U) can be administered to the target person (T) over a long period of time (one hour or more). In this embodiment, the predetermined time is set to one hour, but the predetermined time may be set to another length depending on the symptom of the target person (T).

Here, the cumulative total of time during which the useful substance (U) is emitted from the emission unit (30) means the cumulative total of time during which the emission unit (30) performs emission operation. For example, even if the emission unit (30) stops emission operation due to the target person (T) waking up during the operation of the emission device (10), the time of emission operation performed before the target person (T) wakes up and the time of emission operation performed after the target person (T) goes back to sleep again are summed to calculate the cumulative total of time.

If the cumulative total of time during which the predetermined useful substance (U) is emitted from the emission unit (30) is more than or equal to the predetermined time in one day (YES in step ST8), the controller (80) stops the operation of the emission device (10). If the cumulative total of time during which the predetermined useful substance (U) is emitted from the emission unit (30) is less than the predetermined time in one day (NO in step ST8), the controller (80) returns to step ST1.

### <Control of Environment Adjustment Unit>

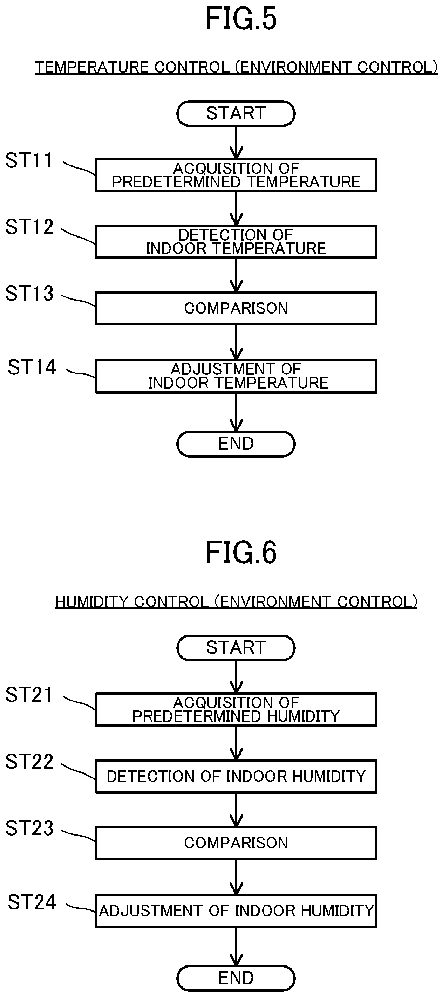

The control of the environment adjustment unit (90) in step ST1 of FIG. 4 will be described with reference to FIGS. 5 to 7. The environment adjustment unit (90) performs at least one of temperature control, humidity control, or airflow control in the indoor space (S). When two or more controls among the temperature control, the humidity control, and the airflow control are performed, the controls may be performed in any order.

### (Temperature Control)

As illustrated in FIG. 5, the controller (80) acquires a predetermined temperature associated with the predetermined useful substance (U) based on the data stored in the storage (82) (step ST11). The predetermined temperature in this embodiment is 23°C or more. Next, the controller (80) detects a temperature of the indoor space (S) based on output from the temperature sensor (51) (step ST12). Next, the controller (80) compares the predetermined temperature acquired from the data in the storage (82) with the temperature of the indoor space (S) (step ST13).

Next, based on the result of the comparison in step ST13, the controller (80) controls the environment adjustment unit (90) so that the temperature of the indoor space (S) reaches a predetermined temperature (step ST14). Specifically, the controller (80) makes the air conditioning unit (20) adjust the temperature of the air in the indoor space (S) to the predetermined temperature. Thus, the temperature of the indoor space (S) can be adjusted to the temperature at which the target person (T) easily absorbs the predetermined useful substance (U).

### (Humidity Control)

As illustrated in FIG. 6, the controller (80) acquires a predetermined humidity associated with the predetermined useful substance (U) based on the data stored in the storage (82) (step ST21). The predetermined humidity in this embodiment is 65%RH. Next, the controller (80) detects a humidity of the indoor space (S) based on an output from the humidity sensor (52) (step ST22). Then, the controller (80) compares the predetermined humidity acquired from the data in the storage (82) with the humidity of the indoor space (S) (step ST23).

Next, based on the result of the comparison in step ST23, the controller (80) controls the environment adjustment unit (90) so that the humidity of the indoor space (S) reaches a predetermined humidity (step ST24). Specifically, the controller (80) makes the air conditioning unit (20) adjust the humidity of the indoor space (S) to the predetermined humidity. Thus, the humidity of the indoor space (S) can be adjusted to the humidity at which the target person (T) easily absorbs the predetermined useful substance (U).

### (Airflow Control)

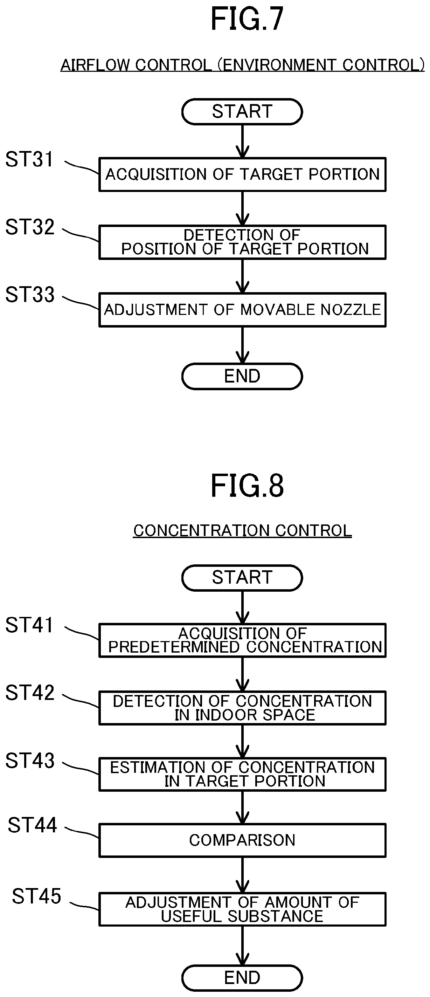

As illustrated in FIG. 7, the controller (80) acquires target portions associated with the predetermined useful substance (U) based on the data stored in the storage (82) (step ST31). Next, the controller (80) detects positions of the target portions of the target person (T) by the position sensor (70) (step ST32). Specifically, the controller (80) detects positions of the mouth and nose of the target person (T) from the information in the camera (55).

Next, the controller (80) adjusts the direction of the movable nozzle (46) of the vortex ring generation unit (31) so that the predetermined useful substance (U) is emitted toward the target portions of the target person (T) (step ST33). In this embodiment, the movable nozzle (46) is adjusted to be directed toward the mouth and nose of the target person (T). Thus, even if the target person (T) turns over in sleep to change its posture for example, the movable nozzle (46) can follow the movement of the target portions.

### <Concentration Control Based on Output from Concentration Sensor>

The concentration control based on the output from the concentration sensor (53) in step ST2 of FIG. 4 will be described with reference to FIG. 8.

As illustrated in FIG. 8, the controller (80) acquires a predetermined concentration associated with a predetermined useful substance (U) based on the data stored in the storage (82) (step ST41). Next, the controller (80) detects a concentration of the useful substance (U) in the indoor space (S) at the position of the concentration sensor (53) based on the output from the concentration sensor (53) (Step ST42). Next, the controller (80) estimates a concentration of the useful substance (U) at the target portion of the target person (T) based on the detected concentration in the indoor space (S) (Step ST43). Next, the controller (80) compares the predetermined concentration acquired from the data in the storage (82) with the estimated concentration at the target portion (step ST44).

Next, based on the result of the comparison in step ST44, the controller (80) adjusts the amount of the useful substance (U) supplied from the substance supply unit (32) so that the estimated concentration reaches the predetermined concentration (step ST45). Thus, the concentration of the useful substance (U) supplied to the target person (T) can be maintained at a predetermined concentration. In addition, the useful substance (U) of which the amount has been adjusted for the target person (T) can be supplied, and thus the amount of the useful substance (U) to be used can be reduced.

### -Features of First Embodiment-

The feature (1) of this embodiment is that the emission device (10) includes the emission unit (30), the environment adjustment unit (90), the storage (82), and the controller (80); the storage (82) stores the data including the useful substance (U) and the environmental conditions associated with the useful substance (U); and the controller (80) acquires the environmental conditions associated with the useful substance (U) emitted from the emission unit (30), based on the data stored in the storage (82), and controls the environment adjustment unit (90) to meet the environmental conditions.

According to this configuration, the information on the environmental conditions in which the useful substance (U) efficiently acts on the target person (T) is stored in the storage (82), whereby the useful substance (U) can be efficiently administered to the target person (T).

The feature (2) of this embodiment is that the controller (80) adjusts the emission amount of the predetermined useful substance (U) based on the output from the concentration sensor (53). According to this configuration, the concentration of the predetermined useful substance (U) supplied to the target person (T) can be maintained at an appropriate concentration. In addition, the useful substance (U) of which the amount has been adjusted for the target person (T) can be supplied, and thus the amount of the useful substance (U) to be used can be reduced.

The feature (3) of this embodiment is that the controller (80) acquires a temperature of the indoor space (S) associated with the predetermined useful substance (U), based on the data stored in the storage (82), and controls the environment adjustment unit (90) to meet that temperature. According to this configuration, the temperature of the indoor space (S) can be adjusted to a temperature at which the useful substance (U) easily acts on the target person (T).

The feature (4) of this embodiment is that the controller (80) acquires a humidity of the indoor space (S) associated with the predetermined useful substance (U), based on the data stored in the storage (82), and controls the environment adjustment unit (90) to meet that humidity. According to this configuration, the humidity of the indoor space (S) can be adjusted to the humidity at which the useful substance (U) easily acts on the target person (T).

The feature (5) of this embodiment is that the controller (80) acquires an airflow in the indoor space (S) associated with the predetermined useful substance (U), based on the data stored in the storage (82), and controls the environment adjustment unit (90) to meet that the airflow. According to this configuration, the airflow in the indoor space (S) can be adjusted to an airflow at which the useful substance (U) easily acts on the target person (T).

The feature (6) of this embodiment is that the environment adjustment unit (90) emits an airflow in a vortex ring shape. According to this configuration, the airflow in a vortex ring shape (vortex ring (R)) is less diffused and has high directivity, and thus, the useful substance (U) applied to the vortex ring (R) can be efficiently administered to the target person (T).

Further, even if two or more persons are present in the indoor space (S), the useful substance (U) can be administered to the target person (T) only. Thus, the useful substance (U) is not administered to a person other than the target person (T), whereby the burden on the person other than the target person (T) is also reduced. In addition, the useful substance (U) is emitted only around the target portion of the target person (T), and thus the useful substance (U) less adheres to a wall or clothes, thereby reducing coloring thereon.

The feature (7) of this embodiment is that the biosensor (60) detects a respiratory cycle of the target person (T), and the controller (80) makes the emission unit (30) emit the useful substance (U) at the time when the target person (T) inhales air. According to this configuration, the useful substance (U) is emitted at the time when the target person (T) inhales air, and thus the amount of the useful substance (U) used can be reduced.

The feature (8) of this embodiment is that the controller (80) makes the emission unit (30) emit the useful substance (U) so that the cumulative total of time during which the useful substance (U) is emitted from the emission unit (30) is one hour or more in one day.

Here, for inhalers used to administer a drug for respiratory diseases and the like, existing concepts of administration for oral drugs are used to determine the concentration, spray volume, spray time, and number of inhalations of the useful substance. In particular, regarding the spray time, spraying is performed in a short time such as about minutes to several tens of minutes. **In** contrast, a person can obtain a high effect of the useful substance when the concentration of the useful substance in the body is maintained at an effective level over a long period of time. Therefore, typical inhalers may not cause the useful substance (U) to be efficiently administered to the target person (T).

According to this configuration, the useful substance (U) can be administered to the target person (T) over a long period of time, and thus the useful substance (U) can be efficiently administered to the target person (T). **In** addition, the useful substance (U) can be administered to the target person (T) over a long period of time, and thus some types of useful substances (U) can be administered at lower concentrations than those at which they are administered over a short period of time. Thus, side effects of the useful substance (U) can be reduced.

### -Variations of First Embodiment-

The first embodiment may be modified as follows. The configurations described in the following variations can also be applied to the second and third embodiments which will be described in detail later.

### <First Variation>

An emission device (10) of this embodiment may use another useful substance as a predetermined useful substance (U) for a disease of asthma. The useful substance (U) in this variation includes cineole, menthol, and terpinen-4-ol. The concentration of the useful substance (U) in this variation is 0.1 ppb to 1000 ppb.

**In** this variation, the emission device (10) may include a contact sensor (54). The contact sensor (54) is a wearable sensor integral with a wristwatch. The contact sensor (54) of this variation is put on an arm of a target person (T). The contact sensor (54) detects signals that underlie the heart rate and body temperature of the target person (T). **In** other words, the contact sensor (54) serves as a biosensor (60) that acquires biological information of the target person (T).

A controller (80) performs control so that an emission unit (30) emits the useful substance (U) at a timing at which the symptom of the target person (T) is likely to appear or at a timing at which the useful substance (U) is likely to act on the target person (T), based on the biological information (information on the heart rate and body temperature) detected by the biosensor (60).

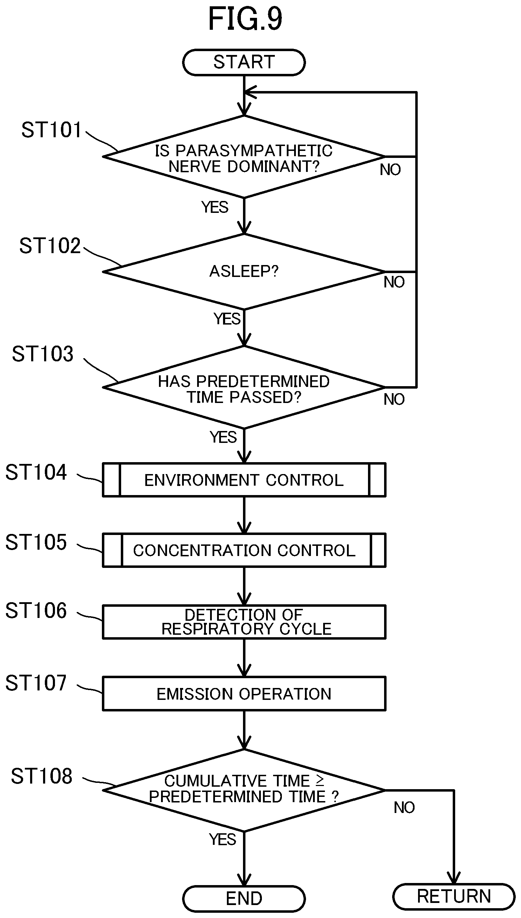

Specifically, as illustrated in FIG. 9, in control of the emission device (10), when the emission device (10) turns into an operating state, the controller (80) determines whether or not the parasympathetic nerve of the target person (T) is dominant (step ST101). Specifically, the controller (80) acquires information on the RR interval of the heart rate from the biosensor (60) in the contact sensor (54) to determine whether or not the parasympathetic nerve of the target person (T) is dominant.

If the parasympathetic nerve of the target person (T) is dominant (YES in step ST101), the controller (80) determines whether or not the target person (T) is asleep (step ST102). Specifically, the controller (80) acquires information on the heart rate and the body temperature from the biosensor (60) in the contact sensor (54) to determine whether or not the target person (T) is asleep. Accordingly, it can be determined that the target person (T) who is asleep and whose parasympathetic nerve is dominant is likely to get a cough attack.

If the target person (T) is asleep (YES in step ST102), the controller (80) determines whether or not the current time has passed a predetermined time (step ST103). In this variation, the predetermined time is midnight. Thus, the useful substance (U) can be administered to the target person (T), before he/she gets a cough attack, who is likely to get a cough attack at dawn (e.g., around 4 a.m.). In this variation, the predetermined time is set to midnight, but the predetermined time may be set to another time depending on the symptom of the target person (T).

If the current time has passed the predetermined time (YES in step ST103), the controller (80) controls the environment adjustment unit (90) (step ST104). The operation after the control of the environment adjustment unit (90) is the same as that in the first embodiment. The predetermined time in step ST5 of this variation is four hours. Thus, the predetermined useful substance (U) can be administered to the target person (T) over a long period of time (one hour or more). In this variation, the predetermined time is set to four hours, but the predetermined time may be set to another length depending on the symptom of the target person (T).

Thus, the controller (80) determines a state of the target person (T) based on the biological information of the target person such that the useful substance (U) can be emitted at an optimum timing for the target person (T) such as a timing at which the symptom of the target person (T) is likely to appear or a timing at which the useful substance (U) is likely to act on the target person (T). Consequently, the useful substance (U) can be efficiently administered to the target person (T).

### <Second Variation>

An emission device (10) of this embodiment may be used for a target person (T) whose disease is allergic rhinitis such as pollinosis.

A useful substance (U) in this variation is levocabastine (corticosteroid). The concentration of the useful substance (U) is 0.0025%. The information of the environmental conditions associated with the useful substance (U) (levocabastine) stored in a storage (82) includes a temperature of 23°C or more, humidity of 65%RH or more, and airflow directed toward the nose.

In the temperature control of an environment adjustment unit (90), the controller (80) makes an air conditioning unit (20) adjust the temperature of the air in an indoor space (S) to 23°C or more. In the humidity control, the controller (80) causes the air conditioning unit (20) to adjust the humidity of the air in the indoor space (S) to 65%RH or more. In the airflow control, the controller (80) adjusts the direction of a movable nozzle (46) of a vortex ring generation unit (31) toward the nose of the target person (T).

In this manner, the controller (80) adjusts the indoor space (S) to an optimum environment where the target person (T) ingests the useful substance (U). Thus, the useful substance (U) can be efficiently administered to the target person (T). In addition, the useful substance (U) in this variation is as effective as the normal nasal drop in a spray volume smaller than that of the normal nasal drop (specifically, about 1/4 of the amount used normally). Therefore, the burden on the target person (T) can be reduced.

### <Third Variation>

An emission device (10) of this embodiment may be used for a target person (T) whose disease is pneumonia (bacterial inflammation).

A useful substance (U) in this variation is gentamicin (antibacterial agent). The concentration of the useful substance (U) is 0.1%. The information of the environmental conditions associated with the useful substance (U) (gentamicin) stored in a storage (82) includes a temperature of 23°C or more, humidity of 65%RH or more, and airflow directed toward the mouth and nose.

In the temperature control of an environment adjustment unit (90), the controller (80) makes an air conditioning unit (20) adjust the temperature of the air in an indoor space (S) to 23°C or more. In the humidity control, the controller (80) causes the air conditioning unit (20) to adjust the humidity of the air in the indoor space (S) to 65%RH or more. In the airflow control, the controller (80) adjusts the direction of a movable nozzle (46) of a vortex ring generation unit (31) toward the mouth and nose of a target person (T).

In this manner, the controller (80) adjusts the indoor space (S) to an optimum environment where the target person (T) ingests the useful substance (U). Thus, the useful substance (U) can be efficiently administered to the target person (T).

### <Fourth Variation>

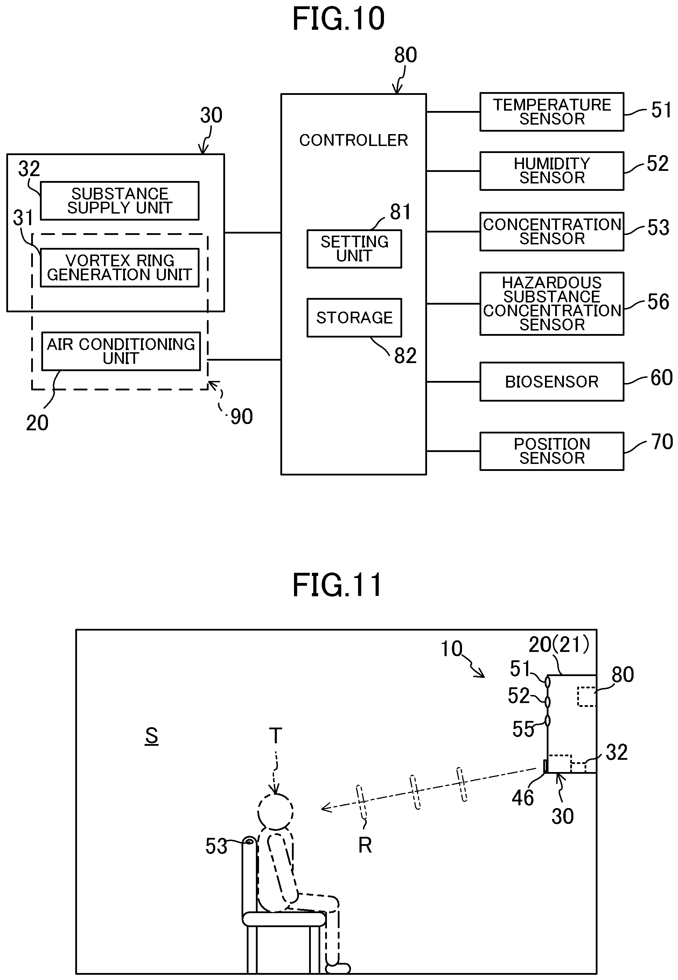

As illustrated in FIG. 10, an emission device (10) of this embodiment may include a hazardous substance concentration sensor (56). Specifically, the hazardous substance concentration sensor (56) measures the concentrations of hazardous substances in the air. The hazardous substances mentioned herein include viruses (e.g., influenza viruses), volatile organic compounds (VOCs), and the like.

The hazardous substance concentration sensor (56) in this variation is attached to an emission unit (30) and exposed to an indoor space (S). The hazardous substance concentration sensor (56) detects the concentrations of hazardous substances in the indoor space (S). The hazardous substance concentration sensor (56) may be provided in an air conditioning unit (20), or may be provided in the indoor space (S) independently of the air conditioning unit (20) or the emission unit (30).

A storage (82) of a controller (80) in this variation stores data including a plurality of types of hazardous substances, useful substances (U) associated with the hazardous substances, and environmental conditions associated with the useful substances (U). The controller (80) is connected to the hazardous substance concentration sensor (56) in a wired or wireless manner, and is able to transmit and receive signals to and from that component. The controller (80) makes the emission unit (30) emit the useful substance (U) based on the output from the hazardous substance concentration sensor (56).

In control of the emission device (10), when the emission device (10) turns into an operating state, the controller (80) determines whether or not the concentration of each of the hazardous substances in the indoor space (S) is more than or equal to a predetermined concentration. Specifically, the controller (80) acquires an output from the hazardous substance concentration sensor (56) to determine whether or not the concentration of each of the hazardous substances in the indoor space (S) is more than or equal to the predetermined concentration. If the concentration of the hazardous substance in the indoor space (S) is more than or equal to the predetermined concentration, the controller (80) selects a useful substance (U) associated with the hazardous substance in the storage (82). Next, the controller (80) controls an environment adjustment unit (90). The operation after the control of the environment adjustment unit (90) is the same as that in the first embodiment.

Thus, if the concentration of a predetermined hazardous substance present in the indoor space (S) is more than or equal to a predetermined concentration, the air conditioning unit (20) adjusts the environmental conditions of the indoor space (S) to environmental conditions where a predetermined useful substance (U) acts efficiently, and the emission unit (30) emits the predetermined useful substance (U) associated with the hazardous substance. Consequently, the useful substance (U) can be efficiently administered to the target person (T).

### <Fifth Variation>

An emission device (10) of this embodiment may include an air purifier (91) instead of an air conditioning unit (20). The air purifier (91) includes an air purification unit (92) and a humidity adjustment unit (93). The air purification unit (92) purifies air in an indoor space (S). The humidity adjustment unit (93) adjusts the humidity of air in the indoor space (S). In this variation, the air purifier (91) serves as an environment adjustment unit (90).

The controller (80) makes the air purification unit (92) purify air in the indoor space (S). In that case, if the emission device (10) includes a hazardous substance concentration sensor (56), the controller (80) makes the air purification unit (92) purify air in the indoor space (S) so that an output from the hazardous substance concentration sensor (56) is less than or equal to a predetermined concentration. Based on the humidity data stored in the storage (82), the controller (80) controls the humidity adjustment unit (93) so that the indoor space (S) has a predetermined humidity.

In control of the emission device (10), if a target person (T) selects his/her symptom or disease, the selected symptom or disease is set in a setting unit (81) of the controller (80), and the emission device (10) starts to operate. In this variation, the target person (T) selects asthma as a disease.

If the disease is asthma, the controller (80) makes the air purification unit (92) purify air in the indoor space (S) when the emission device (10) turns into an operating state. Specifically, the controller (80) makes the air purification unit (92) remove microparticles from the air in the indoor space (S) in order to purify the air. Here, the microparticles to be removed are preferably allergic substances which cause development of asthmatic.

Next, the controller (80) stops purification of the air purification unit (92). Then, the controller (80) controls an environment adjustment unit (90). Specifically, the controller (80) acquires a predetermined humidity associated with a predetermined useful substance (U), based on the data stored in the storage (82), and makes the humidity adjustment unit (93) adjust the humidity so that the air in the indoor space (S) has the predetermined humidity. The operation after the control of the environment adjustment unit (90) is the same as that in the first embodiment.

In this way, after the air purification unit (92) purifies air in the indoor space (S) and removes allergic substances that cause symptoms, the emission unit (30) emits a prescribed useful substance (U). Thus, the useful substance (U) can be more efficiently administered to the target person (T), and the symptoms can be alleviated. In addition, after the air purification unit (92) stops its operation, the emission unit (30) emits the predetermined useful substance (U). This avoids purification of the emitted useful substance (U) by the air purification unit (92).

Further, if the target person (T) has a disease that is allergic rhinitis (pollinosis), the microparticles removed by the air purification unit (92) are preferably allergic substances such as pollen, exhaust gas particles, or yellow dust. If the target person (T) has a symptom of pneumonia (bacterial inflammation), the microparticles removed by the air purification unit (92) are preferably microparticles that irritate a trachea of the target person (T), or VOCs.

### <<Second Embodiment>>

A second embodiment will be described. An emission device (10) of this embodiment is configured such that an emission unit (30) and an air conditioning unit (20) in the emission device (10) of the first embodiment are integral with each other. Thus, the following description will be focused on the differences of the emission device (10) of this embodiment from the emission device (10) of the first embodiment.

As illustrated in FIG. 11, a target person (T) sits on a chair in an indoor space (S), for example. The emission unit (30) of the emission device (10) is provided in an indoor unit (21) of the air conditioning unit (20). Specifically, a vortex ring generation unit (31) and a substance supply unit (32) are provided in the indoor unit (21). An emission port (44) of the vortex ring generation unit (31) is provided at a blow-out port of the indoor unit (21).

A vortex ring (R) generated by the vortex ring generation unit (31) is emitted from the blow-out port of the indoor unit (21) toward a target portion of the target person (T). In other words, the air conditioning unit (20) serves as the emission unit (30) and also serves as an environment adjustment unit (90).

The air conditioning unit (20) adjusts environmental conditions of the indoor space (S). Specifically, the air conditioning unit (20) adjusts a temperature, humidity, and airflow direction of air in the indoor space (S). A controller (80) acquires the environmental conditions (a temperature, humidity, and airflow direction) associated with the useful substance (U) stored in the storage (82), and controls the air conditioning unit (20) to meet the environmental conditions.

A camera (55) as a biosensor is attached to the indoor unit (21). The camera (55) is directed toward the indoor space (S) and captures an image of the indoor space (S). A concentration sensor (53) is disposed above the chair on which the target person (T) sits.

Also in this embodiment, the controller (80) acquires the environmental conditions associated with to the useful substance (U) emitted from the emission unit (30), based on the data stored in the storage (82), and controls the environment adjustment unit (90) to meet the environmental conditions. Thus, the information on the environmental conditions in which the useful substance (U) efficiently acts on the target person (T) is stored in the storage (82), whereby the useful substance (U) can be efficiently administered to the target person (T).

### <<Third Embodiment>>

A third embodiment will be described below. An emission device (10) of this embodiment is configured such that an emission unit (30) in the emission device (10) of the first embodiment includes a sprayer (38) of an electrostatic spray type instead of a vortex ring generation unit (31). Thus, the following description will be focused on the differences of the emission device (10) of this embodiment from the emission device (10) of the first embodiment.

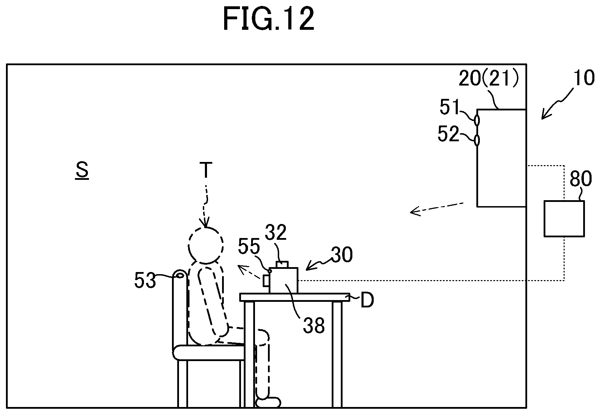

As illustrated in FIG. 12, a target person (T) sits on a chair in an indoor space (S), for example. The emission device (10) is disposed on a desk (D) placed in front of the target person (T). The emission unit (30) of the emission device (10) includes the sprayer (38) of an electrostatic spray type. The sprayer (38) includes a spray nozzle and a counter electrode. The counter electrode is disposed around the spray nozzle. In this embodiment, the substance supply unit (32) stores a liquid useful substance (U).

In this embodiment, when a high voltage is applied to the spray nozzle and the outside, a potential difference is generated between charged liquid and the counter electrode at the distal end of the spray nozzle, and an electric field is generated. The liquid on the distal end of the spray nozzle is pulled by the electric field and ejected in a liquid thread state. Afterward, the liquid ejected in a liquid thread state splits into fine droplets. The liquid is charged and repulse each other such that the droplets are diffused and sprayed.

According to this configuration, the useful substance (U) emitted from the emission device (10) can be made into fine particles by the sprayer (38) of an electrostatic spray type. Thus, the useful substance (U) can be efficiently administered to the target person (T). In addition, the useful substance (U) emitted from the sprayer (38) of an electrostatic spray type is charged, and thus the useful substance (U) is attracted to a target portion of the target person (T) serving as a ground. Thus, the useful substance (U) can be sprayed and concentrated on the target portion of the target person (T).

Also in this embodiment, the controller (80) acquires the environmental conditions associated with to the useful substance (U) emitted from the emission unit (30), based on the data stored in the storage (82), and controls the environment adjustment unit (90) to meet the environmental conditions. Thus, the information on the environmental conditions in which the useful substance (U) efficiently acts on the target person (T) is stored in the storage (82), whereby the useful substance (U) can be efficiently administered to the target person (T).

### <<Other Embodiments>>

The above embodiments may also have the following configurations.

The emission unit (30) of the emission device (10) of the first and third embodiments is placed on furniture (a shelf and a desk) in a room, but may be of a floor-mounted type where the emission unit (30) is installed on a floor surface or may be a wall-mounted type where the emission unit (30) is installed on a wall surface. Further, the emission device (10) of the first and third embodiments may be installed on a ceiling.

The air conditioning unit (20) of each of the embodiments may be a humidifier configured to humidify an indoor space.

The emission device (10) of each of the embodiments may include an emission unit (30) having both a vortex ring generation unit (31) and a sprayer (38).

The emission unit (30) of the third embodiment may be integral with an air conditioning unit (20).

The vortex ring generation unit (31) of the emission unit (30) of the first embodiment may not necessarily include the extrusion mechanism (45) having the linear actuator (45b) and the diaphragm (45a). For example, the extrusion mechanism (45) may be a piston that reciprocates to convey air in the air passage (C).

In the emission device (10) of each of the embodiments, the adjustment of the airflow by the environment adjustment unit (90) may be performed in a way using other than the vortex ring generation unit (31). For example, an airflow direction in the indoor unit may be adjusted to make an airflow in the indoor space (S) adjusted.

The controller (80) of each of the embodiments performs emission of the useful substance (U) when the target person (T) is asleep or works at the desk, but may perform emission of the useful substance (U) during a period of time in which the useful substance (U) can be efficiently administered (e.g., during watching of a movie, reading, eating, or the like) depending on a symptom of the target person (T).

The controller (80) of each of the embodiments performs emission of the useful substance (U) toward the mouth and nose, but may perform emission toward other target portions depending on a disease or symptom of the target person (T). Examples of the other target portions include skin, a head (scalp and hair root), and eyes (cornea and conjunctiva).

The emission device (10) of each of the embodiments may not include the biosensor (60). Specifically, the camera (55) may not detect a respiratory cycle of the target person (T). In that case, the emission unit (30) emits the predetermined useful substance (U) without synchronization with the respiratory cycle of the target person (T).

The camera (55) of each of the embodiments may detect biological information (e.g., a heart rate, a pulse wave, or a facial color) other than the respiratory cycle of the target person (T) depending on the symptom.

The contact sensor (54) of the first variation of the embodiment may detect biological information (e.g., blood pressure, blood sugar, skin moisture, or body motion) other than a body temperature and a heart rate depending on the symptom.

The embodiments, the variations, and the other embodiments may be combined and replaced with each other without deteriorating intended functions of the present disclosure.

### INDUSTRIAL APPLICABILITY

As described above, the present disclosure is useful for an emission device.

### DESCRIPTION OF REFERENCE CHARACTERS

- 10: Emission Device
- 30: Emission Unit
- 31: Vortex Ring Generation Unit
- 38: Sprayer
- 53: Concentration Sensor
- 80: Controller
- 82: Storage
- 90: Environment Adjustment Unit
- T: Target Person
- S: Indoor Space (Space)
- U: Useful Substance

## Claims

1. An emission device, comprising:
an emission unit (30) configured to emit a useful substance (U) to a space (S) where a target person (T) is present;
an environment adjustment unit (90) configured to adjust an environmental condition of the space (S); **characterized in that** the emission device further comprises
a storage (82) configured to store data on the useful substance (U) and an environmental condition associated with the useful substance (U); and
a controller (80) configured to acquire the environmental condition associated with the useful substance (U) emitted from the emission unit (30), based on the data stored in the storage (82), and control the environment adjustment unit (90) to meet the environmental condition.

2. The emission device of claim 1, further comprising:
a concentration sensor (53) configured to measure a concentration of the useful substance (U) supplied to the target person (T), wherein
the controller (80) adjusts an emission amount of the useful substance (U) based on an output from the concentration sensor (53).

3. The emission device of claim 1 or 2, wherein
the environment adjustment unit (90) adjusts a temperature of air in the space (S), and
the controller (80) acquires a temperature of the space (S) associated with the useful substance (U), based on the data stored in the storage (82), and controls the environment adjustment unit (90) to meet the temperature.

4. The emission device of any one of claims 1 to 3, wherein
the environment adjustment unit (90) adjusts a humidity of air in the space (S), and
the controller (80) acquires a humidity of the space (S) associated with the useful substance (U), based on the data stored in the storage (82), and controls the environment adjustment unit (90) to meet the humidity.

5. The emission device of any one of claims 1 to 4, wherein
the environment adjustment unit (90) adjusts an airflow in the space (S), and
the controller (80) acquires an airflow of the space (S) associated with the useful substance (U), based on the data stored in the storage (82), and controls the environment adjustment unit (90) to meet the airflow.

6. The emission device of any one of claims 1 to 5, wherein
the environment adjustment unit (90) emits an airflow in a vortex ring shape.

## Patentansprüche

1. Abgabevorrichtung, umfassend:
eine Abgabeeinheit (30), die ausgebildet ist, einen Nutzstoff (U) an einen Raum (S) abzugeben, in dem sich eine Zielperson (T) befindet;
eine Umgebungsanpassungseinheit (90), die ausgebildet ist, eine Umgebungsbedingung des Raums (S) anzupassen; **dadurch gekennzeichnet, dass** die Abgabevorrichtung weiter umfasst,
einen Speicher (82), der ausgebildet ist, Daten über den Nutzstoff (U) und eine dem Nutzstoff (U) zugeordnete Umgebungsbedingung zu speichern; und
eine Steuereinheit (80), die ausgebildet ist, die dem von der Abgabeeinheit (30) abgegebenen Nutzstoff (U) zugeordnete Umgebungsbedingung basierend auf den in dem Speicher (82) gespeicherten Daten zu erfassen und die Umgebungsanpassungseinheit (90) zu steuern, um die Umgebungsbedingung zu erfüllen.

2. Abgabevorrichtung nach Anspruch 1, weiter umfassend:
einen Konzentrationssensor (53), der ausgebildet ist, eine Konzentration des der Zielperson (T) zugeführten Nutzstoffs (U) zu messen, wobei
die Steuereinheit (80) eine Abgabemenge des Nutzstoffs (U) basierend auf einer Ausgabe des Konzentrationssensors (53) anpasst.

3. Abgabevorrichtung nach Anspruch 1 oder 2, wobei
die Umgebungsanpassungseinheit (90) eine Temperatur von Luft in dem Raum (S) anpasst, und
die Steuereinheit (80) eine dem Nutzstoff (U) zugeordnete Temperatur des Raums (S) basierend auf den in dem Speicher (82) gespeicherten Daten erfasst und die Umgebungsanpassungseinheit (90) steuert, um die Temperatur zu erfüllen.

4. Abgabevorrichtung nach einem der Ansprüche 1 bis 3, wobei
die Umgebungsanpassungseinheit (90) eine Feuchtigkeit von Luft in dem Raum (S) anpasst, und
die Steuereinheit (80) eine dem Nutzstoff (U) zugeordnete Feuchtigkeit des Raums (S) basierend auf den in dem Speicher (82) gespeicherten Daten erfasst und die Umgebungsanpassungseinheit (90) steuert, um die Feuchtigkeit zu erfüllen.

5. Abgabevorrichtung nach einem der Ansprüche 1 bis 4, wobei
die Umgebungsanpassungseinheit (90) einen Luftstrom in dem Raum (S) anpasst, und
die Steuereinheit (80) einen dem Nutzstoff (U) zugeordneten Luftstrom des Raums (S) basierend auf den in dem Speicher (82) gespeicherten Daten erfasst und die Umgebungsanpassungseinheit (90) steuert, um den Luftstrom zu erfüllen.

6. Abgabevorrichtung nach einem der Ansprüche 1 bis 5, wobei
die Umgebungsanpassungseinheit (90) einen Luftstrom in einer Wirbelringform abgibt.

## Revendications

1. Dispositif d'émission, comprenant :
une unité d'émission (30) configurée pour émettre une substance utile (U) vers un espace (S) où une personne cible (T) est présente ;
une unité de réglage d'environnement (90) configurée pour régler une condition environnementale de l'espace (S) ; **caractérisé en ce que** le dispositif d'émission comprend en outre
une mémoire (82) configurée pour stocker des données sur la substance utile (U) et une condition environnementale associée à la substance utile (U) ; et
un dispositif de commande (80) configuré pour acquérir la condition environnementale associée à la substance utile (U) émise par l'unité d'émission (30), sur la base des données stockées dans la mémoire (82), et commander l'unité de réglage d'environnement (90) pour satisfaire la condition environnementale.

2. Dispositif d'émission selon la revendication 1, comprenant en outre :
un capteur de concentration (53) configuré pour mesurer une concentration de la substance utile (U) fournie à la personne cible (T), dans lequel
le dispositif de commande (80) règle une quantité d'émission de la substance utile (U) sur la base d'une sortie provenant du capteur de concentration (53).

3. Dispositif d'émission selon la revendication 1 ou la revendication 2, dans lequel
l'unité de réglage d'environnement (90) règle une température d'air dans l'espace (S), et
le dispositif de commande (80) acquiert une température de l'espace (S) associée à la substance utile (U), sur la base des données stockées dans la mémoire (82), et commande l'unité de réglage d'environnement (90) pour satisfaire la température.

4. Dispositif d'émission selon une quelconque des revendications 1 à 3, dans lequel
l'unité de réglage d'environnement (90) règle une humidité d'air dans l'espace (S), et
le dispositif de commande (80) acquiert une humidité de l'espace (S) associée à la substance utile (U), sur la base des données stockées dans la mémoire (82), et commande l'unité de réglage d'environnement (90) pour satisfaire l'humidité.

5. Dispositif d'émission selon une quelconque des revendications 1 à 4, dans lequel
l'unité de réglage d'environnement (90) règle un flux d'air dans l'espace (S), et
le dispositif de commande (80) acquiert un flux d'air de l'espace (S) associé à la substance utile (U), sur la base des données stockées dans la mémoire (82), et commande l'unité de réglage d'environnement (90) pour satisfaire le flux d'air.

6. Dispositif d'émission selon une quelconque des revendications 1 à 5, dans lequel
l'unité de réglage d'environnement (90) émet un flux d'air présentant une forme d'anneau tourbillonnaire.
